# EUROPÄISCHE PATENTANMELDUNG

(11) **EP 1 464 334 A1**
(43) Veröffentlichungstag der Anmeldung: **06.10.2004**
(21) Anmeldenummer: 03007421.5
(22) Anmeldetag: 03.04.2003
(51) Int. Cl.: A61K 31/47, A61L 2/00, A61L 2/16, A61L 2/18

(54) **Dekontaminationsmittel gegen Prionen**

(71) Anmelder: Fluka GmbH, 9471 Buchs (CH)
(72) Erfinder: Gabriel, Edwin, 6020 Emmenbrücke (CH); Wahl, Fabian, Dr., 9470 Buchs (CH)
(74) Vertreter: Keller, Günter, Dr.

(57) **Zusammenfassung**

Die Erfindung betrifft ein Dekontaminationsmittel gegen Prionen, das wenigstens eine tricyclische Verbindung mit einem Substituenten an Position 9 enthält. Weiterhin wird ein Verfahren zur Dekontamination von Gegenständen, insbesondere von medizinischen Geräten, bereitgestellt.

## Beschreibung

Die vorliegende Erfindung betrifft das Gebiet der Dekontamination und Sterilisierung von Gegenständen, insbesondere von medizinischen Instrumenten und Analysegeräten. Es wird ein Dekontaminationsmittel zur Verfügung gestellt, das es ermöglicht, Gegenstände zu dekontaminieren, die mit infektiösen Prionen verunreinigt sind. Insbesondere werden Verbindungen zur Verfügung gestellt, die als Dekontaminationsmittel gegen Prionen geeignet sind.

Prionkrankheiten sind prinzipiell übertragbare, tödlich verlaufende neurodegenerative Erkrankungen. Allen Prionkrankheiten ist gemeinsam, dass der relativen kurzen klinischen Krankheitsphase eine lange Inkubationszeit vorausgeht. Pathophysiologisch wird in einem noch nicht vollständig geklärten autokatalytischen Prozeß das physiologische, auf Nervenzellen vorkommende membranständige Prionprotein in seiner Tertiärstruktur verändert. Dabei entsteht die sogenannte Scrapie-Isoform des Prionproteins (PrP^{Sc}), die angereichert wird und für den Körper schwer abbaubar ist. Entsprechend der Prionhypothese ist die Scrapie-Isoform des Prionproteins selbst Teil des infektiösen Agens oder mit diesem identisch. Die Scrapie-Isoform selbst kann eine Konformationsänderung des zellulären, natürlich vorkommenden Prionproteins induzieren.

Bekannte Prionkrankheiten bei Tieren sind "Scrapie" (Schafe und Ziegen), "bovine spongiform encephalopathy", kurz BSE (Rinder) und "feline spongiform encephalopathy" (Katzen). Prionkrankheiten beim Menschen können idiopathisch, hereditär oder als akquirierte Erkrankung auftreten:
- Die idiopathische Kreutzfeldt-Jakob-Erkrankung, auch als sporadische oder klassische CJD bezeichnet, ist eine rasch fortschreitende dementielle Erkrankung mit einer Krankheitsdauer von etwa vier bis sechs Monaten, die vorwiegend im sechsten oder siebten Lebensjahrzehnt auftritt. In Deutschland sind 85 bis 90% der Kreutzfeldt-Jakob-Erkrankungen zu dieser idiopathischen Form zu rechnen.
- Hereditären Prionkrankheiten (familiäre CJD = fCJD) gehen mit einer autosomal dominant vererbten Mutation im Prionprotein-Gen einher. Hierzu zählen auch das Gerstmann-Sträussler-Scheinker-Syndrom (GSS) und die tödliche familiäre Insomnie (fatal familial insomnia = FFI).
- Zu den akquirierten Prionerkrankungen gehören iatrogene Erkrankungen in Folge der Verabreichung von Hypophysen-Hormonpräparaten, die aus Leichenhypophysen gewonnen wurden, Dura mater- und Cornea-Transplantationen sowie eine erstmals 1996 beschriebene Variante der Kreutzfeldt-Jakob-Erkrankung (vCJD), die mit sehr großer Wahrscheinlichkeit durch Übertragung des BSE-Erregers auf den Menschen zustande kommt und bislang nur in England, Frankreich und Nordirland gesehen worden ist.

Erreger übertragbarer spongiformer Enzephalopathien ("transmissible spongiform enzephalopathies" = TSE) sind extrem widerstandsfähig und werden durch die herkömmlichen Desinfektions- und Sterilisationsmaßnahmen nicht inaktiviert. Selbst Röntgenstrahlen werden bis 25 KGy toleriert, und selbst bei 600°C trockener Heißlufteinwirkung bleibt bei sehr hohem Ausgangstiter eine Restinfektiosität übrig. Bisher werden folgende Maßnahmen zur Dekontamination von Instrumenten/Materialien gegen Prionen angewendet:

Arbeitsflächen werden durch Exposition mit 2 M NaOH dekontaminiert. NaOH ist jedoch nicht gut auf Aluminium- oder Zinnoberflächen zu verwenden. Mitunter wird anstelle von NaOH auch die Verwendung von Natriumhypochlorit empfohlen. Natriumhypochlorit korrodiert Metalle und sollte nicht auf Oberflächen verwendet werden, da sich reizende Gase bilden können. Verschiedentlich wurde auch das Einlegen medizinischer Instrumente in 3 bis 6 M GdnSCN für bis zu 24 Stunden oder das Kochen der Geräte in 3% Natriumdodecylsulfat (SDS) empfohlen.

Materialien, die dampfsterilisierbar sind, können bei 134°C für 1 Stunde oder bei 121°C für 4,5 Stunden dampfautoklaviert werden. Es gibt vollautomatische Programme zur Dampfsterilisation von Instrumentarium, Flüssigkeiten und zur sogenannten Vernichtungssterilisation. Die Abluft dieser Geräte wird über einen Sterilfilter mit einer Porengröße von 0,2 µm abgeführt, wobei der Filter bei jedem Sterilisiervorgang mitsterilisiert wird.

Die genannten Maßnahmen haben den Nachteil, dass sie aufwendig sind und/oder den Einsatz von aggressiven, zum Teil gefährlichen Substanzen erfordern.

WO 00/72851 A1 offenbart ein Verfahren zur Sterilisierung von Gegenständen, bei dem kontaminierte Gegenstände mit einem polykationischen Dendrimer in Kontakt gebracht werden

Doh-Ura et al. (2000) Journal of Virology 74, 4894-4897 offenbart, dass lysosomotrope Stoffe und Cysteinprotease-Inhibitoren die Anreicherung von Prion-Protein in ScNB-Zellen hemmen. Die Autoren sind der Ansicht, dass die Hemmung nicht auf eine direkte Wechselwirkung mit Prion-Protein zurückzuführen ist, sondern möglicherweise durch Änderung des lysosomalen pH erfolgt.

Korth et al. (2001) Proceedings of the National Academy of Sciences USA 98, 9836-9841 beschreibt, dass Acridin- und Phenothiazin-Derivate die Bildung von Prion-Protein in ScN2a-Zellen hemmen. Die Autoren betonen, dass es keine Hinweise auf eine direkte Wechselwirkung von Chinacrin und Prion-Protein gibt.

May et al. (2003) Proceedings of the National Academy of Sciences USA 100, 3416-3421 offenbart, dass bis-Acridine die Replikation von Scrapie-Prionen in kultivierten Zellen hemmen. Es wird angenommen, dass die bis-Acridine an einen Rezeptor auf der Zelloberfläche binden, der an der Bildung von Prion-Protein beteiligt ist.

Eine Aufgabe der vorliegenden Erfindung ist es, ein vorteilhaftes Dekontaminationsmittel gegen Prionen bereitzustellen.

Überraschenderweise wurde gefunden, dass planare tricyclische Ringsysteme mit einem Substituenten in Position 9 auch außerhalb von Zellen anti-Prion-Aktivität aufweisen. Die Erfindung betrifft daher die Verwendung einer Verbindung der Formel I 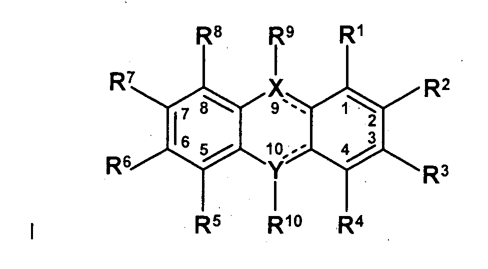 wobei R⁹ eine gegebenenfalls substituierte aromatische Gruppe oder eine gegebenenfalls substituierte nicht-aromatische Gruppe ist, R¹, R², R³, R⁴, R⁵, R⁶, R⁷, R⁸ und R¹⁰ jeweils gleich oder verschieden und unabhängig voneinander Wasserstoff, ein Halogenatom, eine Hydroxylgruppe, eine gegebenenfalls substituierte aromatische oder nicht aromatische Gruppe ist, mit der Maßgabe, dass R¹⁰ nichts ist, wenn Y Stickstoff oder Schwefel ist, X Kohlenstoff, Stickstoff oder Schwefel ist, Y Kohlenstoff, Stickstoff oder Schwefel ist, oder von Salzen davon als Dekontaminationsmittel gegen Prionen.

Der Substituent R⁹ ist mit dem tricyclischen Ringsystem in Position 9 verbunden und stellt eine aromatische oder nicht-aromatische, z.B. eine aliphatische Gruppe dar. R⁹ ist nicht Wasserstoff. Es ist bevorzugt, dass R⁹ eine nicht-aromatische, z.B. eine aliphatische Gruppe ist. Die nicht-aromatische Gruppe kann eine unsubstituierte oder substituierte Kohlenstoffkette mit einer Länge von 1 bis 25, bevorzugt 2 bis 15, bevorzugter 3 bis 12, am bevorzugtesten 4 bis 10, insbesondere 8 Atomen sein, wobei 0 bis 5 der Atome in der Kohlenstoffkette Heteroatome sein können. Vorzugsweise sind 0 bis 4 Atome in der Kohlenstoffkette Heteroatome, bevorzugter 1 bis 3, am bevorzugtesten 2. Heteroatome im Sinne der vorliegenden Erfindung sind insbesondere Sauerstoff, Stickstoff und/oder Schwefel. Es ist bevorzugt, dass das Heteroatom Stickstoff ist. In einer besonderen Ausführungsform ist das Atom der Kohlenstoffkette, das unmittelbar mit dem Ringatom 9 des tricyclischen Ringsystems verbunden ist, ein Stickstoffatom. Die Länge der Kohlenstoffkette ist die Anzahl der Atome in der unverzweigten Kette. Im Fall von Verzweigungen und/oder Substitutionen der Kohlenstoffkette ist die Länge die Anzahl der längsten möglichen linearen Kette, ohne die Verzweigungen und/oder Substituenten der Kohlenstoffkette mitzuzählen.

Ein nicht-aromatischer Substituent R⁹, z.B. eine Kohlenstoffkette wie oben beschrieben, kann gesättigt oder ungesättigt sein. Liegt ein ungesättigter Substituent R⁹ vor, so kann er eine oder mehrere Doppelbindungen enthalten. Die Anzahl der Doppelbindungen in einer Kohlenstoffkette R⁹ ist üblicherweise 0 bis 10, vorzugsweise 0 bis 5, bevorzugter 0 bis 3, am bevorzugtesten 0 bis 2. Üblicherweise handelt es sich um C=C-Doppelbindungen, es können aber auch N=C-Doppelbindungen sein. Es ist ebenfalls möglich, dass der Substituent eine oder mehrere Dreifachbindungen enthält. In der Regel liegt nicht mehr als eine Dreifachbindung vor. Bei der Dreifachbindung handelt es sich in der Regel um eine C≡C-Dreifachbindung. Die ungesättigten Bindungen können in der Kohlenstoffkette oder möglichen Substituenten davon enthalten sein.

Die Gruppe R⁹ kann auch Verzweigungen und/oder Substitutionen aufweisen. Bevorzugt ist es, wenn eine Kohlenstoffkette, wie sie oben beschrieben wurde, 1 bis 4 Substitutionen aufweist, am bevorzugtesten 2 oder 3. Die Substitutionen können von C-Atomen oder N-Atomen der Kohlenstoffkette ausgehen. Mögliche Substituenten sind Halogenatome, Hydroxyl-, Alkyl-, Alkenyl-, Alkinyl-, Alkoxy-, Acyl-, Aryl-, Amino-, Amido-, Imino-, Oxo-, Ester-, Nitro-, Sulfonyl- und/oder Carboxylgruppen, ohne dass diese Aufzählung abschließend ist. Die Alkyl-, Alkenyl-, Alkinyl,- Alkoxy- oder Acylgruppe kann 1 bis 20 C-Atome, vorzugsweise 1 bis 15 C-Atome, bevorzugter 1 bis 10 C-Atome, am bevorzugtesten 1 bis 5 C-Atome aufweisen. Bevorzugt kommen als Substituenten Alkylgruppen vor, insbesondere Methyl- oder Ethyl- oder Propylgruppen. Am bevorzugtesten sind Methyl- oder Ethylgruppen. Ist der Substituent eine Arylgruppe, so kann diese auch heteroaromatisch sein. Sie kann 5 bis 24, vorzugsweise 5 bis 18, bevorzugter 5 bis 12, am bevorzugtesten 5 oder 6 C-Atome umfassen und substituiert oder unsubstituiert sein. Die Arylgruppe kann 1 bis 3, vorzugsweise einen oder zwei, aromatische Ringe umfassen. Die übrigen genannten Substituenten, insbesondere die Amino-, Amido-, Imino-, Ester-, Nitro- oder Sulfonylgruppen können mit Alkyl-, Alkenyl- oder Alkinylgruppen mit 1 bis 20 C-Atomen, vorzugsweise 1 bis 15 C-Atomen, bevorzugter 1 bis 10 C-Atomen, am bevorzugtesten 1 bis 5 C-Atomen substituiert sein.

Vorzugsweise ist R⁹ eine gesättigte Kohlenstoffkette mit einer Länge von 1 bis 25, bevorzugt 2 bis 15, bevorzugter 3 bis 12, am bevorzugtesten 4 bis 10, insbesondere 8 Atomen, wobei die Kohlenstoffkette 0 bis 3 Alkylsubstituenten mit 1 bis 5 C-Atomen aufweist und 0 bis 3 Stickstoffatome enthält.

Beispiele für nicht-aromatische Gruppen R⁹ sind eine Dimethylaminopropyl- oder eine Dimethylamino-2-methylpropyl-Seitenkette. Am bevorzugtesten ist der Substituent R eine [4-(Diethylamino)-1-Methylbutyl-] Aminogruppe.

Ist R⁹ eine aromatische Gruppe, so kann diese auch heteroaromatisch sein. Sie kann 5 bis 24, bevorzugt 5 bis 18, bevorzugter 5 bis 12, am bevorzugtesten 5 oder 6 C-Atome umfassen und substituiert oder unsubstituiert sein. Mögliche Substituenten entsprechen den oben beschriebenen. Die aromatische Gruppe kann 1 bis 3, vorzugsweise einen oder zwei, aromatische Ringe umfassen.

Das Atom X im zentralen Ring ist Kohlenstoff, Stickstoff oder Schwefel. Wenn X Kohlenstoff ist, ist die gestrichelte Linie in der allgemeinen Formel I, die X mit dem benachbarten C-Atom verbindet, eine chemische Bindung. Diese Bindung ist in der Regel nicht vorhanden, wenn X Stickstoff ist. Das Ringatom Y kann Kohlenstoff, Stickstoff oder Schwefel sein. Vorzugsweise ist Y Stickstoff. Die gestrichelte Linie, die eine chemische Bindung von Y zum Nachbaratom darstellt, ist üblicherweise nicht vorhanden, wenn Y Schwefel ist. Sie ist vorhanden, wenn Y Kohlenstoff oder Stickstoff ist. Wenn Y Kohlenstoff ist, weist es in der Regel eine Bindung zu einem Wasserstoffatom auf (R¹⁰ = H). Wenn Y Stickstoff oder Schwefel ist, ist R¹⁰ in der Regel nichts.

Die Reste R¹, R², R³, R⁴, R⁵, R⁶, R⁷, R⁸ und R¹⁰ können jeweils gleich oder verschieden und unabhängig voneinander Wasserstoff, ein Halogenatom, eine Hydroxylgruppe, eine gegebenenfalls substituierte aromatische Gruppe oder eine gegebenenfalls substituierte nicht-aromatische Gruppe sein. Die Reste R¹ bis R⁸ und R¹⁰ können jeweils gleich oder verschieden und unabhangig voneinander beispielsweise ein Wasserstoffatom, eine Hydroxylgruppe, Chlor, Brom, lod, eine Alkyl-, Alkenyl-, Alkinyl-, Alkoxy- oder Acylgruppe mit jeweils 1 bis 20 C-Atomen, vorzugsweise mit 1 bis 15 C-Atomen, bevorzugter mit 1 bis 10 C-Atomen, am bevorzugtesten mit 1 bis 5 C-Atomen sein. R' bis R⁸ und R¹⁰ können weiterhin jeweils gleich oder verschieden und unabhängig voneinander eine Amino-, Amido-, Imino-, Oxo-, Ester-, Nitro-, Sulfonyl- und/oder Carboxylgruppe sein. Liegt eine Aminogruppe vor, so kann diese mit einer Alkyl-, Alkenyl- oder Alkinylgruppe mit 1 bis 20 C-Atomen, vorzugsweise 1 bis 15 C-Atomen, bevorzugter 1 bis 10 C-Atomen, am bevorzugtesten 1 bis 5 C-Atomen substituiert sein. Auch die weiteren Gruppen können mit diesen Alkyl-, Alkenyl- oder Alkinylgruppen substituiert sein. Liegt ein Arylsubstituent vor, so umfasst dieser in der Regel 5 bis 24, vorzugsweise 5 bis 18, bevorzugter 5 bis 12, am bevorzugtesten 5 oder 6 C-Atome. Es kann sich auch um einen Heteroarylrest handeln. Weiterhin kann ein Cycloalkyl oder Heterocycloalkylsubstituent vorliegen. Ebenfalls ist es möglich, dass verschiedene Substituenten einen oder mehrere Ringe bilden, die an das tricyclische Ringsystem ankondensiert sind.

Vorzugsweise ist R² nicht ein Wasserstoffatom. Bevorzugt ist R² eine Acyl- oder eine Alkoxygruppe, am bevorzugtesten eine Acetyl- oder eine Methoxygruppe.

Es ist auch bevorzugt, dass R⁶ kein Wasserstoffatom ist. Bevorzugter ist R⁶ ein Halogenatom, insbesondere Chlor.

In einer besonderen Ausführungsform sind R² und R⁶ kein Wasserstoffatom. Dabei kann R ² eine Alkoxygruppe, insbesondere eine Methoxygruppe, und R⁶ ein Halogenatom, insbesondere ein Chloratom, sein.

Es ist bevorzugt, dass R¹, R³, R⁴, R⁵, R⁷, R⁸ und R¹⁰ Wasserstoffatome sind.

Als Dekontaminationsmittel gegen Prionen können beispielsweise die Verbindungen Promazin (II), Chlorpromazin (III), Acepromazin (IV) oder Promethazin (V) eingesetzt werden, deren Formeln nachstehend wiedergegeben sind:

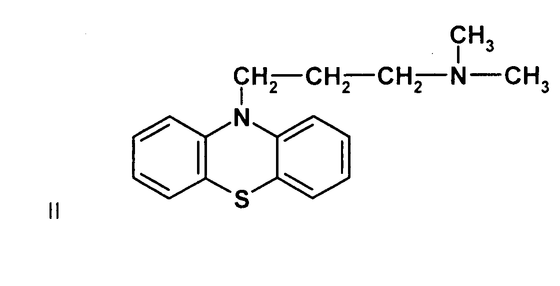

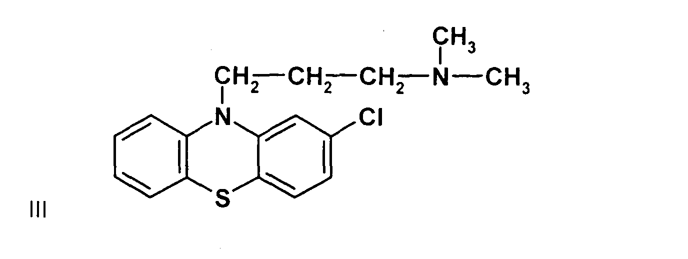

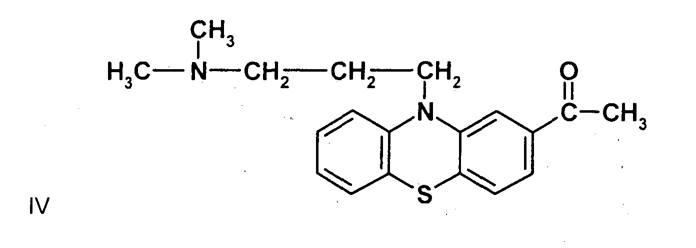

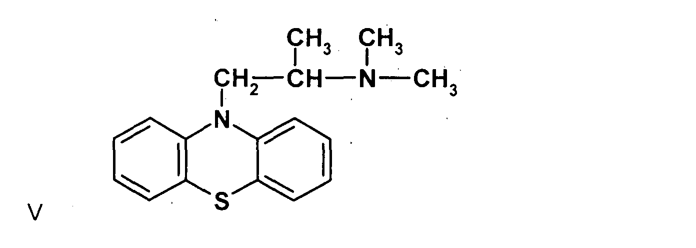

Am bevorzugtesten wird Chinacrin oder ein Salz davon eingesetzt. Chinacrinhydrochlorid ist seit mehr als 60 Jahren als Medikament weltweit registriert und wird heute noch eingesetzt Die Hauptindikationsgebiete sind die nicht-invasive Sterilisation von Frauen in Entwicklungsländern, Therapie von Malaria, Protozoenerkrankungen (Giardiose), rheumatischen Erkrankungen und pleuraler Sklerose. Bei allen Indikationen werden orale Tagesdosen von bis zu 1000 mg über längere Zeiträume verabreicht und von den Patienten toleriert. Die LD₅₀-Dosis von Chinacrinhydrochlorid wird mit 660 mg/kg (oral Ratte), 557 mg/kg (oral Maus) bzw. 433 mg/kg (oral Kaninchen) angegeben (siehe JPETAB 91, 157, 1947). Chinacrin hat folgende Struktur (VI):

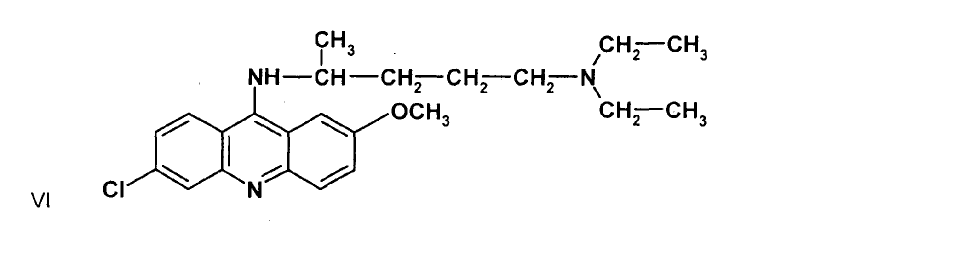

Verfahren zur Synthese von Verbindungen der Formeln I bis VI sind dem Fachmann bekannt. Beispielsweise offenbart US 2,113,357 ein Verfahren zur Synthese von Chinacrin.

Weitere Verbindungen, die erfindungsgemäß eingesetzt werden können, sind bis-Acridine, wie sie in May et al. (2003) Proceedings of the National Academy of Sciences USA 100, 3416-3421 beschrieben sind. Bevorzugt sind insbesondere die bis-Acridin-Analoga (6-Chlor-2-methoxy-acridin-9-yl)-(3-{4-[3-(6-chlor-2-methoxy-acridin-9-ylamino)-propyl]-piperazin-1-yl}-propyl)-amin, *N,N'*-bis-(6-Chlor-2-methoxy-acridin-9-yl)-1 ,8-diamino-3,6-dioxaoctan und (1-{[4-(6-Chlor-2-methoxy-acridin-9-ylamino)-butyl]-[3-(6-chlor-2-methoxyacridin-9-ylamino)-propyl]-carbamoyl}-ethyl)-carbaminsäure-tert-butylester.

Die Verbindungen der Formel I können in jeglicher Form vorliegen, bevorzugt als Lösung. Geeignete Lösungsmittel für die Verbindungen der Formel I sind Wasser, Ethanol, Methanol, Propylenglykol und Dipropylenglykol. Die Zusammensetzung, die die Verbindung der Formel I enthält, hat in der Regel einen pH von 1 bis 13, vorzugsweise von 3 bis 11, am bevorzugtesten von 4 bis 5, z.B. 4,5.

Ein Gegenstand, der im Verdacht steht, Prionen zu enthalten, kann dekontaminiert werden, indem der Gegenstand mit einer Verbindung der Formel I in Kontakt gebracht wird. Der Begriff "Dekontamination" im Sinne dieser Anmeldung bedeutet die Umwandlung von einem möglicherweise infektiösen Zustand in einen nicht-infektiosen Zustand. Insbesondere ist damit die Umwandlung eines in seiner Konformation geänderten Proteins (z.B. PrP^{Sc}, bekannt als Prionen) in einen Zustand gemeint, der nicht mehr infektiös ist und keine Prionkrankheit mehr auslösen kann.

Eine Zusammensetzung, die eine Verbindung der Formel I enthält, wird als Dekontaminationsmittel eingesetzt. In erster Linie können Gegenstände wie chirurgische Instrumente, Elektroden für invasive Hirnuntersuchungen, Endoskopie, sowie Geräte und Analysensysteme für die klinisch-chemische und molekularbiologische Analytik und andere Geräte und Instrumente dekontaminiert werden. Die Erfindung betrifft aber auch die Dekontamination von biologischen Zusammensetzungen, z.B. Zellhomogenaten, Gewebeschnitten, Fleisch etc. Im Rahmen der vorliegenden Erfindung umfasst der Begriff "Gegenstand" auch derartige biologische Proben, nicht aber lebende Menschen oder Tiere. Diese Dekontamination von Gegenständen ist somit keine therapeutische Behandlung des lebenden menschlichen oder tierischen Körpers.

Ein anderer Aspekt der Erfindung ist aber die Dekontamination von Haut von Personen oder Tieren, die mit potentiell infektiösem Material in Kontakt gekommen sind. Die Erfindung betrifft daher auch die Verwendung einer Verbindung der Formel I zur Herstellung eines Mittels zur Dekontamination von möglicherweise kontaminierter Haut. Erfindungsgemäß wird dabei das Dekontaminationsmittel nur äußerlich und/oder topisch angewendet, nicht systemisch verfügbar gemacht.

Das Dekontaminationsmittel kann auf einem zu dekontaminierenden Gegenstand verstrichen werden oder darauf gesprüht werden. In einer besonderen Ausführungsform wird der zu dekontaminierende Gegenstand in eine Lösung des Dekontaminationsmittels eingetaucht. Diese Ausführungsform kann bevorzugt bei medizinischen Geräten wie Skalpellen, Klammern, Elektroden, etc. eingesetzt werden. Gemäß einer anderen Ausführungsform wird der zu dekontaminierende Gegenstand von dem Dekontaminationsmittel durchströmt. Dafür kommen insbesondere Meßinstrumente und Analysegeräte in Frage, die Röhren oder Schläuche umfassen, durch die potentiell Prionenhaltiges Material geflossen ist. Das Dekontaminationsmittel kann einfach durch diese Schläuche oder Röhren gepumpt oder gesaugt werden. Es ist weiterhin vorteilhaft, wenn das Dekontaminationsmittel in Form eines Sprays oder Schaums angewendet werden kann. Dazu wird das Dekontaminationsmittel vorzugsweise in einem Behältnis bereitgestellt, das unter Druck steht. Durch Zugabe bestimmter Treibmittel oder Schäummittel kann eine Abgabe aus dem Behältnis in feinverteilter Form oder als Schaum erzielt werden. Dem Fachmann sind die Verfahren bekannt, durch die Sprays oder Schäume bereitgestellt werden können. Derartige Verfahren sind beispielsweise in International Cosmetic Ingredient Dictionary and Handbook, 9th Ed. 2002 (CTFA) erläutert.

Ein weiterer Aspekt ist die Verwendung des erfindungsgemäßen Dekontaminationsmittels in Schlachtbetrieben oder Metzgereien, um potentiell kontaminierte Gerätschaften zu dekontaminieren. Dazu zählen u.a. Schlachtwerkzeug, Messer, Arbeitsflächen, etc.

Der zu dekontaminierende Gegenstand wird für einen ausreichenden langen Zeitraum mit dem Dekontaminationsmittel in Kontakt gebracht, so dass eine effektive Dekontamination erreicht wird. Der Zeitraum beträgt in der Regel 1 Minute bis 1 Woche, vorzugsweise 10 Minuten bis 24 Stunden, am bevorzugtesten 1 Stunde bis 12 Stunden. Dem Fachmann ist bewusst, dass der Zeitraum von der Konzentration der wirksamen Verbindung in dem Dekontaminationsmittel und von dessen Temperatur abhängt.

Die Konzentration der Verbindung der Formel I in dem Dekontaminationsmittel wird so gewählt, dass eine effektive Dekontamination des Gegenstands erreicht werden kann. Übliche Konzentrationen sind 0,001 mM bis 1 M, vorzugsweise 0,01 mM bis 100 mM, am bevorzugtesten 0,05 bis 10 mM. Dem Fachmann ist bewusst, dass eine längere Dekontaminationszeit eine geringere Konzentration ausgleichen kann und dass bei gegebenenfalls kürzerer Dekontaminationszeit eine höhere Konzentration gewählt werden muß.

Das Dekontaminationsmittel kann weiterhin andere Verbindungen enthalten, beispielsweise Verbindungen, die eine antibakterielle, antivirale und/oder anti-Prion-Wirkung haben. Beispiele für derartige zusätzliche Verbindungen, die enthalten sein können, sind Säuren (organische und anorganische); Alkalien; Alkohole (Ethanol, Isopropylalkohol); Aldehyde (Glyoxal, Formaldehyd, Glutaraldehyd); Biguanide (Chlorhexidin); chloraktive Verbindungen (Natriumhypochlorit); lodophore; phenolische Desinfektionsmittel (2-Biphenylol, p-Chlor-m-kresol); Peroxide; quaternäre Ammoniumverbindungen; Amphotenside; kationische Tenside; Guanidiniumhydrochlorid und/oder Guanidiniumrhodanid.

In einer besonderen Ausführungsform wird das Dekontaminationsmittel in erhitzter Form mit dem zu dekontaminierenden Gegenstand in Kontakt gebracht. Bevorzugte Temperaturen liegen im Bereich von 30 bis 70°C, am bevorzugtesten im Bereich von 40 bis 60°C, insbesondere ungefähr 50°C.

Ein weiterer Gegenstand der Erfindung ist ein Verfahren zur Dekontamination eines Gegenstands, der möglicherweise Prionen enthält, wobei der Gegenstand mit einer Verbindung der Formel I in Kontakt gebracht wird. Die bevorzugten Ausführungsformen des Verfahrens entsprechen den oben beschriebenen bevorzugten Ausführungsformen.

Ein weiterer Aspekt der Erfindung ist ein Dekontaminationsmittel gegen Prionen, das dadurch gekennzeichnet ist, dass es eine Verbindung der Formel I enthält. Die bevorzugten Ausführungsformen des Dekontaminationsmittels, insbesondere hinsichtlich der darin enthaltenen Verbindung der Formel I, entsprechen den oben beschriebenen bevorzugten Ausführungsformen. Vorzugsweise enthält das Dekontaminationsmittel neben einer Verbindung der Formel I wenigstens eine weitere Verbindung, die antibakterielle, antivirale und/oder anti-Prion-Wirkung aufweist. Bevorzugte weitere Verbindungen, die neben einer Verbindung der Formel I enthalten sein können, sind obenstehend beschrieben. In einer anderen Ausführungsform enthält das erfindungsgemäße Dekontaminationsmittel wenigstens zwei verschiedene Verbindungen der Formel I.

Die Erfindung betrifft weiterhin einen Kit zur Dekontamination von Gegenständen umfassend ein derartiges Dekontaminationsmittel. Vorzugsweise enthält das Kit das Dekontaminationsmittel in Form einer Lösung, die in einem Behältnis angeordnet ist. Dabei kann es sich um ein Gefäß handeln, aus dem das Dekontaminationsmittel in Form eines Sprays oder Schaums abgegeben werden kann. In einer anderen Ausführungsform enthält der Kit die Verbindung der Formel I in konzentrierter Form, entweder als Feststoff oder als konzentrierte Lösung oder Suspension. Zur Durchführung der Kontamination kann dann die gebrauchsfertige Dekontaminationslösung einfach durch Verdünnung oder Lösen hergestellt werden. Der Kit kann weitere Verbindungen, die zur Sterilisation geeignet sind, wie z.B. antivirale und/oder antibakterielle Verbindungen enthalten.

Die verschiedenen bevorzugten Ausführungsformen, wie sie oben beschrieben worden sind, können beliebig miteinander kombiniert werden. Derartige Kombinationen sind von der vorliegenden Erfindung umfasst.

Die vorliegende Erfindung stellt ein Dekontaminationsmittel gegen Prionen bereit. Dadurch kann es vermieden werden, dass umständliche und aufwendige Prozeduren wie das Autoklavieren/Dampfsterilisieren angewendet werden müssen. Ebenso kann es vermieden werden, dass aggressive Substanzen wie NaOH oder GdSCN eingesetzt werden müssen. Die Erfindung ermöglicht es daher, Gegenstände auf einfache Weise zu dekontaminieren und von infektiösen Prionen zu befreien.

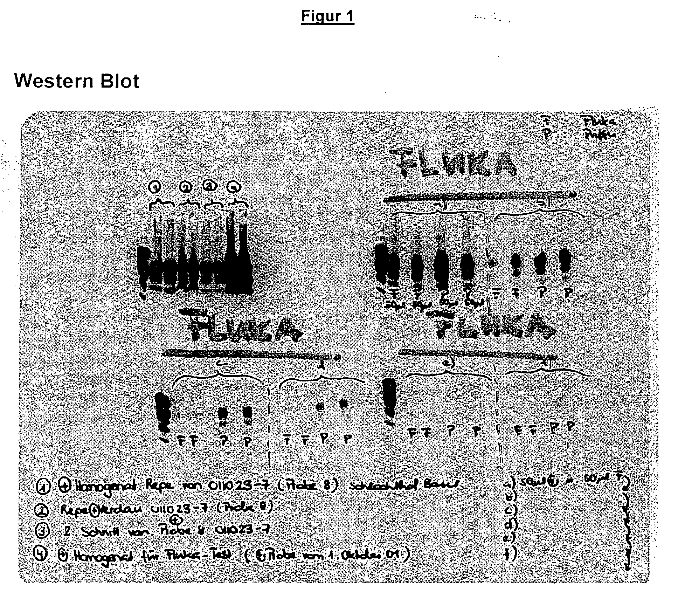

Figur 1 zeigt das Ergebnis eines Western Blots, aus dem hervorgeht, dass durch Zugabe von steigenden Mengen Chinacrinhydrochlorid zu einem Homogenat mit infektiösen Prionen der Anteil an Proteinase K-resistentem Prionprotein stark verringert werden kann (siehe Beispiel 1). Der Ansatz 4 ist das im Test eingesetzte BSE-positive Homogenat.

Die folgenden Beispiele erläutern die Erfindung näher.

### Beispiel 1

Homogenate von verschiedenen BSE-positiven Schlachtproben (Schlachthaus Basel) wurden gemischt und als Positivprobe (PrP^{Sc}) für die Untersuchung der Prionendekontaminationslösung eingesetzt. In Figur 1 ist die Positivprobe Homogenat 4.

Jeweils 50 µl der Positivprobe wurden mit einem steigenden Volumen an 0,1 mM Chinacrindihydrochlorid-Lösung in Wasser (=Dekontaminationslösung) für 16 Stunden bei Raumtemperatur inkubiert. Anschließend wurde ein Western Blot durchgeführt, wie er bei Routineuntersuchungen mit Rinderhirnhomogenat angewendet wird (siehe Schaller O. et al. Acta Neuropathol. (Berl) 1999 (Nov. 98) (5) 437-443). Folgende Ansätze wurden hergestellt:
50 µl Positivprobe (PrP^{sc}) wurden mit 50 µl Dekontaminationslösung gemischt (a).

Von Ansatz (a) wurden 50 µl entnommen und mit weiteren 50 µl Dekontaminationslösung gemischt (b).

Von Ansatz (b) wurden 50 µl entnommen und mit weiteren 50 µl Dekontaminationslösung gemischt (c).

Von Ansatz (c) wurden 50 µl entnommen und mit weiteren 50 µl Dekontaminationslösung gemischt (d).

Von Ansatz (d) wurden 50 µl entnommen und mit weiteren 50 µl Dekontaminationslösung gemischt (e).

Von Ansatz (e) wurden 50 µl entnommen und mit weiteren 50 µl Dekontammationslösung gemischt (f).

Als Kontrolle wurden entsprechende Ansätze hergestellt, wobei anstelle der Dekontaminationslösung ein Puffer eingesetzt wurde, der keine Verbindung der Formel I enthielt.

Nach Inkubation für 16 Stunden bei Raumtemperatur wurden die verschiedenen Ansätze mit Proteinase K behandelt und durch SDS-PAGE untersucht. Wie in Figur 1 gezeigt ist, ist mit steigenden Konzentrationen von Chinacrin eine erhöhte Proteinase K-Sensitivität zu beobachten. Dass auch in der Kontrolle "P" die Banden, die Protease-resistentes Prion-Protein zeigen, schwächer werden, liegt an der Verdünnung, die von Ansatz a) bis f) zunimmt

### Beispiel 2

Die Verbindungen Promazin (II), Chlorpromazin (III), Acepromazin (IV) und Promethazin (V) wurden mit dem in Beispiel 1 dargestellten Verfahren auf ihre Anti-Prion-Aktivität hin untersucht Sie zeigten dabei Anti-Prion-Aktivität, wenn auch geringer als die von Chinacrin (VI).

### Beispiel 3

### Formulierungsbeispiel A:

| **Verbindung** | **Konzentration** |
|---|---|
| Chinacrin-Hydrochlorid | 5 x 10⁻⁵ Gew.-% - 2,9 Gew.-% |
| aktive Biozid-Mischung | 1, 5, 2,0 bzw. 3,0 Gew.-% |
| Stabilisator (Magnesiumsalze, mod. Glycole, bzw. Alkylcarboxylate) | 25 Gew.-% - 90 Gew.-% |
| Wasser | Ad 100 Gew.-% |
| pH 4,5 - 6 | |

### Vergleichsbeispiel

Die Verbindungen Phenazin (VII), Phenothiazin (VIII) und 2-Chlor-Phenothiazin (IX) wurden mit dem in Beispiel 1 dargestellten Verfahren auf ihre Anti-Prion-Aktivitat hin untersucht.

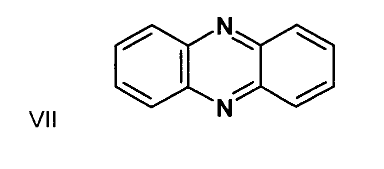

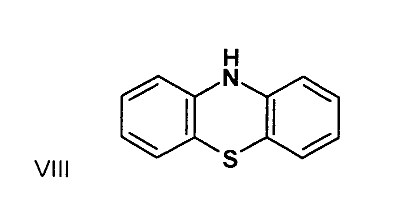

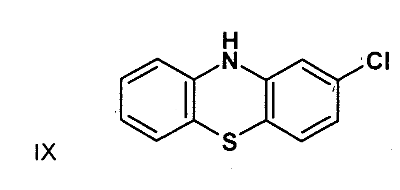

Dabei zeigten die getesteten Verbindungen VII bis IX praktisch keine PrP^{Sc}-Inhibition. Das zeigt, dass das tricyclische Grundgerüst allein für die Anti-Prion-Aktivität nicht ausreicht. Es ist ein Substituent in 9-Stellung erforderlich.

## Patentansprüche

1. Verwendung einer Verbindung der Formel I 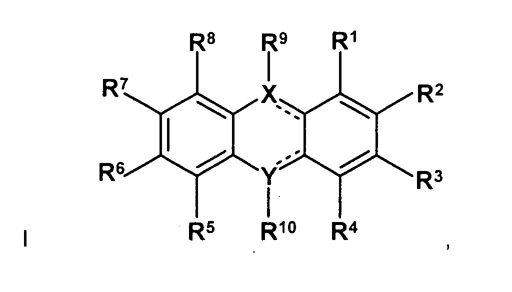 wobei R⁹ eine gegebenenfalls substituierte aromatische oder nicht-aromatische Gruppe ist, R¹ R², R³, R⁴, R⁵, R⁶, R⁷, R⁸ und R¹⁰ jeweils gleich oder verschieden und unabhängig voneinander Wasserstoff, ein Halogenatom, eine Hydroxylgruppe, eine gegebenenfalls substituierte aromatische oder nicht-aromatische Gruppe ist, mit der Maßgabe, dass R¹⁰ nichts ist, wenn Y Stickstoff oder Schwefel ist,
X Kohlenstoff, Stickstoff oder Schwefel ist, und
Y Kohlenstoff, Stickstoff oder Schwefel ist,
oder von Salzen davon als Dekontaminationsmittel gegen Prionen.

2. Verwendung nach Anspruch 1, **dadurch gekennzeichnet, dass** R ausgewählt ist aus der Gruppe bestehend aus einer Dimethylaminopropyl-, einer Dimethylamino-2-Methylpropyl- und einer [4-(Diethylamino)-1-Methylbutyl-] Amino-Gruppe.

3. Verwendung nach Anspruch 1 oder 2, **dadurch gekennzeichnet, dass** die Verbindung der Formel I ausgewählt ist aus der Gruppe bestehend aus Chinacrin, Promazin, Chlorpromazin, Acepromazin und Promethazin.

4. Verwendung nach einem der Ansprüche 1 bis 3, **dadurch gekennzeichnet, dass** Geräte oder Instrumente dekontaminiert werden.

5. Verwendung nach einem der Ansprüche 1 bis 4, **dadurch gekennzeichnet, dass** die Verbindung der Formel I in einer Konzentration von 0,01 mM bis 100 mM eingesetzt wird.

6. Verwendung nach einem der Ansprüche 1 bis 5, **dadurch gekennzeichnet, dass** eine Zusammensetzung umfassend eine Verbindung der Formel I für 10 Minuten bis 24 Stunden mit einem zu dekontaminierenden Gegenstand in Kontakt gebracht wird.

7. Verwendung nach einem der Ansprüche 1 bis 6, **dadurch gekennzeichnet, dass** ein zu dekontaminierender Gegenstand in eine Lösung mit dem Dekontaminationsmittel getaucht wird.

8. Verwendung nach einem der Ansprüche 1 bis 6, **dadurch gekennzeichnet, dass** ein zu dekontaminierender Gegenstand von dem Dekontaminationsmittel durchströmt wird.

9. Verwendung nach einem der Ansprüche 1 bis 6, **dadurch gekennzeichnet, dass** ein zu dekontaminierender Gegenstand mit dem Dekontaminationsmittel besprüht wird.

10. Verwendung nach einem der vorhergehenden Ansprüche, **dadurch gekennzeichnet, dass** die Verbindung der Formel I in Kombination mit wenigstens einer weiteren Verbindung, die antibakterielle, antivirale und/oder anti-Prion-Aktivität aufweist, eingesetzt wird.

11. Verfahren zur Dekontamination eines Gegenstands, der möglicherweise Prionen enthält, wobei der Gegenstand mit einer Zusammensetzung umfassend eine Verbindung, wie sie in einem der Ansprüche 1 bis 3 definiert ist, in Kontakt gebracht wird.

12. Dekontaminationsmittel gegen Prionen, **dadurch gekennzeichnet, dass** es eine Verbindung, wie sie in einem der Ansprüche 1 bis 3 definiert ist, umfasst.

13. Dekontaminationsmittel nach Anspruch 12, **dadurch gekennzeichnet, dass** es wenigstens eine weitere Verbindung, die antibakterielle, antivirale und/oder anti-Prion-Aktivität aufweist, umfasst

14. Kit zur Dekontamination von Gegenständen, umfassend ein Dekontaminationsmittel nach Anspruch 12 oder 13.

15. Kit nach Anspruch 14, **dadurch gekennzeichnet, dass** es ein Dekontaminationsspray enthält
